# EUROPEAN PATENT SPECIFICATION

(11) **EP 2 505 667 B1**
(45) Date of publication and mention of the grant of the patent: **16.07.2014**
(21) Application number: 12152245.2
(22) Date of filing: 24.01.2012
(51) Int. Cl.: C12Q 1/68

(54) **Device and method for analysis of kidney failure**
Vorrichtung und Verfahren zur Analyse von Nierenversagen
Dispositif et procédé pour l'analyse de l'insuffisance rénale

(30) Priority: 03.02.2011 EP 11153273
(43) Date of publication of application: 03.10.2012
(73) Proprietor: Medizinische Hochschule Hannover, 30625 Hannover (DE)
(72) Inventor: Thum, Thomas, Prof. Dr. Dr., 30659 Hannover (DE); Lorenzen, Johan, Dr., 30559 Hannover (DE); Kielstein, Jan, PD Dr., 39112 Magdeburg (DE)
(74) Representative: Taruttis, Stefan Georg

(56) References cited:
- EP-A1- 2 133 431
- EP-A1- 2 474 622
- WO-A1-2010/145035
- US-A1- 2011 003 704
- J. M. LORENZEN ET AL: "Circulating miR-210 Predicts Survival in Critically Ill Patients with Acute Kidney Injury", CLINICAL JOURNAL OF THE AMERICAN SOCIETY OF NEPHROLOGY, vol. 6, no. 7, 23 June 2011 (2011-06-23), pages 1540-1546, XP055044638, ISSN: 1555-9041, DOI: 10.2215/CJN.00430111
- FICHTLSCHERER STEPHAN ET AL: "Circulating microRNAs in patients with coronary artery disease.", CIRCULATION RESEARCH 3 SEP 2010 LNKD- PUBMED:20595655, vol. 107, no. 5, 3 September 2010 (2010-09-03), pages 677-684, XP009150296, ISSN: 1524-4571
- JORDAN YZ LI ET AL: "Review: The role of microRNAs in kidney disease", NEPHROLOGY, vol. 15, no. 6, 1 September 2010 (2010-09-01), pages 599-608, XP055002640, ISSN: 1320-5358, DOI: 10.1111/j.1440-1797.2010.01363.x
- KAUCSAR T ET AL: "Post-transcriptional gene-expression regulation by micro RNA (miRNA) network in renal disease", ADVANCED DRUG DELIVERY REVIEWS, ELSEVIER BV, AMSTERDAM, NL, vol. 62, no. 14, 30 November 2010 (2010-11-30), pages 1390-1401, XP027511629, ISSN: 0169-409X, DOI: DOI:10.1016/J.ADDR.2010.10.003 [retrieved on 2010-11-22]
- BHATT KIRTI ET AL: "microRNAs in kidneys: biogenesis, regulation, and pathophysiological roles", AMERICAN JOURNAL OF PHYSIOLOGY, AMERICAN PHYSIOLOGICAL SOCIETY, BETHESDA, MD, US, vol. 300, no. 3, 12 January 2011 (2011-01-12), pages F602-F610, XP009150142, ISSN: 0002-9513
- YAKLIN KRISTINA M: "Acute kidney injury: an overview of pathophysiology and treatments", NEPHROLOGY NURSING JOURNAL, JANNETTI PUBLICATIONS, INC., PITMAN, NJ, US, vol. 38, no. 1, 1 January 2011 (2011-01-01), pages 13-19, XP009150230, ISSN: 1526-744X

## Description

The present invention relates to an analytical method, and to a kit of parts, which is adapted to the analytical method, for analyzing a patient sample in order to determine the prospects of a patient to develop acute kidney injury (AKI), and to determine the prospects of patients suffering from AKI for long term survival, e.g. for 28 days from the diagnosis of AKI. The invention provides for an analyte, and for the use of the analyte, which in respect to AKI is essentially independent from a diagnosed sepsis or shock. The concentration of the analyte of the invention, preferably in combination with the concentration of one or both of two additional analytes in a sample of human body fluid, e.g. in blood serum obtained from patients, especially from patients suffering from AKI, is a marker of the propensity to develop AKI, and for survival, e.g. of 28-day survival, including renal recovery.

### State of the art

Juan et al. in Urology 835-841 (2010) describe the identification of microRNAs which are characteristic for kidney cancer in comparison to microRNAs from normal kidney.

Mitchell et al., PNAS 10513-10518 (2008) describe circulating microRNAs as markers for cancer, especially of miR-141 as a characteristic for prostate cancer.

Ali et al., J. Am. Soc. Nephrol. 1292-1298 (2007) describe the analysis of creatinine values related to acute kidney injury and acute-on-chronic renal failure.

Bagger et al., Pediatr. Nephrol. 1655-1658 (2007) describe serum creatinine levels and urine output as measures that reflect renal function.

US 2010/0173288 A1 describes that generally disease states can be analysed by measurement of all detectable microRNAs in blood serum for a patient, and describe a kit and biochip, respectively, containing essentially all mature microRNAs known from human serum.

WO 2010/145035 A1 relates to markers for renal diseases and mentions miR-210 as a marker for renal cancer.

US 2011/0003704 A1 describes the analysis of microvesicles for diagnosis of diseases and mentions a very large number of microRNAs, without specifically correlating a specific microRNA to acute kidney failure.

Li et al., Nephrology 599 - 608 (2010) describes that it has been observed in relation to renal carcinoma that miR210 is regulated by hypoxia and may have implications for tumour pathogenesis.

EP 2474622 A1, which is relevant under article 54 (3) EPC only, describes the analysis of increased levels of miR-127, miR-126, miR-210 and miR-101 in comparison to control values, the increase being indicative of acute renal damage samples obtained from patients after kidney transplantation and in patients after cardiac surgery.

### Object of the invention

It is an obj ect of the invention to provide for an analytical method, and for a kit of parts suitable for performing the method, each suitable for evaluating the risk of patients to develop AKI, and suitable for evaluating the risk of mortality, and the prospects of long-term survival and renal recovery, respectively, in AKI patients.

### General description of the invention

The invention achieves the above-mentioned objective by providing a method according to claim 1 for analysis of the status of kidney function in a sample of body fluid obtained from a patient who has not been diagnosed with acute kidney injury (AKI, also referred to as acute kidney failure) or in a sample of body fluid obtained from a patient who has been diagnosed with AKI, which analysis is suitable for and adapted to evaluating the prospects for long-term survival, e.g. for 28 days from the diagnosis of AKI, and/or for renal recovery in an AKI patient, which method for analysis comprises determining the concentration of the microRNA-210 (miR-210) comprising or consisting of the nucleic acid sequence SEQ ID NO 1: CUGUGCGUGUGACAGCGGCUGA, preferably in combination with determining the concentration of one or both of miR-320 comprising or consisting of the nucleic acid sequence SEQ ID NO 2: AAAAGCUGGGUUGAGAGGGCGA and miR-16 comprising or consisting of the nucleic acid sequence SEQ ID NO 3: UAGCAGCACGUAAAUAUUGGCG in a sample, preferably in a serum sample obtained from the patient, which patient can be diagnosed to suffer from AKI, e.g. by analysis of creatinine levels.

The invention is based on the finding that the concentration of the analyte miR-210, preferably in combination with one or both of miR-320 and miR-16, is significantly correlated with the propensity of a patient to initially develop AKI, and with the prospects of survival, e.g. for 28 days, and for renal recovery of AKI patients. In greater detail, it has been found that a serum concentration of miR-210 that is elevated in comparison to median serum levels is indicative of a propensity of a person to develop AKI, and of a reduced prospect for long-term survival of AKI patients, but is generally indicative of an increased mortality. The median serum level can e.g. be the concentration of the respective microRNA found in healthy persons, especially of miR-210s.

Concentrations of miR-320 which are decreased below median serum concentrations of healthy persons are also indicative of an increased propensity to initially develop AKI, indicative of an increased mortality, and indicative of a reduced prospect of long-term survival in AKI patients, and concentrations of miR-16 decreased below a median concentration in healthy persons are also indicative of an increased mortality.

In a first embodiment, the analytical method for determining the concentration of miR-210, optionally in combination with determining the concentration of miR-320 and/or of miR-16, allows the evaluation of the risk of a patient to develop AKI, and/or of the propensity of a patient to suffer from or develop AKI, as the concentration of the analytes of the invention is a predictive indicator for AKI, which indicator is independent from other known indicators for AKI, e.g. from diagnosis of sepsis or shock, and essentially independent from diagnosis of creatinine levels and/or from urine output.

Generally, the method for analysis can be performed by detecting the concentration of the microRNAs which are used as the analyte, i.e. miR-210, preferably additionally miR-320 and/or miR-16 in a sample of body fluid for determining the concentration of the analyte miRNA. The step of determining the concentration of the analyte in a first embodiment generally comprises the step of contacting a sample of body fluid with a nucleic acid sequence which comprises or consists of a section that is reverse complementary to the analyte microRNA (miR), and detecting the concentration or number of hybrids formed by the reverse complementary nucleic acid section with sample components. For detecting the concentration or number of analyte-specific hybrids, it is preferred that the nucleic acid sequence comprising or consisting of a nucleic acid section which is reverse complementary to the analyte miR is immobilized on a substrate, e.g. the nucleic acid sequence can be contained in a panel of nucleic acid sequences which are immobilized and spaced apart on a carrier substrate, such as panels or nucleic acid arrays in the form of a chip.

In a further embodiment, the step of determining the concentration of the one or more analyte miR can be performed by reverse transcription and PCR (RT-PCR) using an oligonucleotide primer which is specific for the analyte miR, followed by reverse transcription and amplification of at least a section of the analyte miR by PCR. For detection, hybridizing probes which are labelled with a chromophor and a spaced apart quencher, the probe having an analyte-specific nucleic acid sequence can be used, e.g. as known for the TaqMan PCR.

Generally the analytical method is performed using an internal control for the reactions of determining the concentration of the analyte miR by adding a known miR as a control to the sample and analysing the concentration of the added control miR by parallel detection with specificity for the control miR. The miR which is added to the sample as an internal control for the analytical method can be a known concentration or copy number of the analyte miR, e.g. a known copy number of miR-210, miR-320 and/or of miR-16, and/or an miR of human or non-human origin, e.g. a synthetic or bacterial microRNA. Preferably, the added internal control miR is produced by bacterial transcription from a DNA sequence encoding the miR, or is produced by enzymatic synthesis, e.g. by PCR, or by chemical synthesis.

The specific amplification of the added internal control miRNA is preferably used for normalization of the determined initial or original serum concentration or copy number of analyte miRNA, e.g. miR-210, miR-320 and/or of miR-16. The initial serum concentration of analyte miRNA was determined by applying a standard curve for the relation of measured amplification of added analyte miRNA of known concentration to the measured number of amplification products obtained in RT-PCR for analyte miRNA. In detail, a standard curve was generated by adding a known copy number (concentration) of analyte miRNA, e.g. of miR-16, miR-210 and miR-320, e.g. obtainable from Ambion Co. at 0.2 fmol/µL analyte miRNA, performing RT-PCR with primers specific for analyte miRNA, and measuring the copy number of specific amplification products. In addition to adding analyte miRNA of known concentration, internal control miRNA, e.g. miR-54 of C. elegans of known copy number and primers specific for internal control miRNA were added. The original serum concentration of analyte miRNA could be calculated by applying the relation of the copy number of analyte-specific amplification products to the original concentration of analyte miRNA, which relation is determined in the standard curve. Normalization could be obtained by dividing the determined concentration of original concentration of analyte miRNA by the concentration of added internal control miRNA.

Accordingly, the method for analysis preferably comprises the calculation of the concentration of the analyte miRNA by means of a standard curve that contains the pre-determined correlation between the concentration of detected analyte miRNA-specific amplification products obtained in RT-PCR to an original (initially known) concentration of analyte miRNA in serum.

In order to account for variability of RNA isolation from serum, the method preferably contains the step of adding a known concentration of a known internal control miRNA to the serum, isolating RNA from the serum, carrying out RT-PCR on the RNA with specific primer pairs added for each of the analyte miRNA and for the control miRNA, respectively, and in addition to calculating the original concentration of analyte miRNA by means of the standard curve, the method also contains the step of calculating the original concentration of added control miRNA by means of a pre-determined standard curve which contains the pre-determined correlation of the detected concentration of amplification products of the control miRNA to the original concentration of control miRNA, and forming the quotient of the calculated original concentration of analyte miRNA and the calculated concentration of control miRNA. Accordingly, it is generally preferred to determine the concentration of analyte miRNA in the serum sample as a quotient to a control miRNA. For example, if control miR-54 of C. elegans was added to 5 fmol/µL serum, for miR-210 as the analyte miRNA, the quotient of (calculated initial concentration of miR-210)/(calculated concentration of control miR-54 (C. elegans)) above 4.00098 x 10⁻⁵ in AKI patients were found to be indicative of a worse prognosis for survival than a lower quotient. The control miRNA is an added miRNA, preferably of a non-human nucleotide sequence, or an miRNA which is determined or known to be present at constant concentrations in human serum.

Preferably, the method of analysis, as well as the determination of the standard curve for analyte miRNA and control RNA, e.g. non-coding RNA, preferably a miRNA, respectively, are carried out using at least 2 different dilutions of the isolated RNA, e.g. 2 to 6 or 3 to 4 dilutions, each e.g. diluted by a factor of 5 to a factor of 10.

In addition to normalization of the analyte miRNA to an added control RNA, e.g. a control miRNA, miR-210 can be normalized to an intrinsic housekeeping RNA, e.g. to a housekeeping small RNA, which preferably is a miRNA or a snoRNA, e.g. the human equivalent to the murine snoRNA202 (SEQ ID NO 5). In these embodiments, concentrations of the analyte miRNA miR-210 are calculated in relation to the concentration of the housekeeping RNA, which is e.g. a snoRNA.

In a further embodiment, the invention relates to the use of nucleic acid sequences containing or consisting of a section that comprises or consists of the nucleic acid sequence of at least one of the analyte microRNAs, and/or to the use of nucleic acid sequences containing or consisting of a section that comprises or consists of a nucleic acid sequence is reverse complementary to at least one of the analyte microRNAs, e.g. to nucleic acid sequences containing or consisting of a nucleotide sequence of miR-210, and/or to nucleic acid sequences containing or consisting of a nucleotide sequence which is reverse complementary to miR-210, optionally additionally to nucleic acid sequences containing or consisting of miR-320, and/or miR-16 and/or nucleic acid sequences containing or consisting of a section that is reverse complementary to miR-320, and/or miR-16, as an indicator substance which is specific for kidney function, especially for use as an indicator substance that is specific for the propensity to suffer from or to develop AKI, and/or as an indicator substance, the concentration of which in a sample of body fluid is indicative of the propensity for an increased mortality for AKI patients.

Optionally, the method includes the step of determining the average concentration of miR-210, optionally additionally of miR-320 and/or of miR-16, in relation to the concentration of a housekeeping microRNA. The concentration of a housekeeping microRNA can be determined by the methods as described for the concentration of miR-210.

### Detailed description of the invention

The invention is now described in greater detail by way of examples with reference to the figures, wherein
- Figure 1 shows the concentration of miR-16 in patients with acute kidney injury (AKI), and in healthy control individuals,
- Figure 2 shows the levels of miR- 320 in AKI patients and in healthy control individuals (CTL),
- Figure 3 shows the concentration of miR-210 in AKI patients and in healthy individuals (CTL),
- Figure 4 shows the concentration of miR-16 in AKI patients grouped for survivors and non-survivors,
- Figure 5 shows the concentration of miR-320 grouped for survivors and non-survivors,
- Figure 6 shows the concentration of miR-210 in AKI patients, grouped for survivors and non-survivors,
- Figure 7 shows the fraction of surviving patients grouped for concentrations of miR-210 below a median concentration, and concentrations above a median concentration, respectively, over the time scale, and
- Figure 8 shows the relative concentration of miR-210 in an acute kidney injury animal experiment.

In the analysis of 77 AKI patients and of 30 healthy age-matched individuals which served as control (CTL), micro-RNAs were quantitatively measured by RT-PCR from serum samples. It was found that in AKI patients, concentrations of miR-210 were higher (p < 0.0001) while concentrations of miR-16 (p < 0.0001) and miR-320 (p < 0.0001) were lower in comparison to healthy individuals. This result shows that a concentration of miR-210 higher than the median concentration in a healthy individual is indicative of AKI or of a significant propensity to suffer from or to develop AKI, whereas concentrations of miR-16 and/or of miR-320 below the median concentration of healthy individuals is also indicative of AKI, or of a significant propensity to suffer from or to develop AKI.

Multivariate Cox regression and Kaplan-Meier curve analyses revealed that the concentration of miR-210 can be used as an independent analyte indicative of the propensity to suffer from or to develop AKI, or for the presence of AKI, as an increased concentration of miR-210 above the median concentration of this analyte in healthy individuals in an independent indicator which is correlated with a reduced 28 - day survival (p = 0.05 and p = 0.03, respectively). Detailed demographic, clinical and laboratory characteristics of a total of 77 patients are shown in Table 1, wherein

APACHE II = acute physiology and chronic health evaluation II, BMI = body mass index, CRP = C-reactive protein, eGFR = estimated glomerular filtration rate; ICU = intensive care unit; MAP = mean arterial blood pressure; n = number of patients; RRT = renal replacement therapy; SOFA = sequential organ failure assessment:

**Table 1: Demographic, clinical and laboratory characteristics of patients**

| | Total | survivors | non-survivors | p-value |
|---|---|---|---|---|
| Number of patients | 77 | 49 | 28 | 0.7 |
| Male (n; %) | 40 (52) | 26 | 14 | |
| Female (n; %) | 37 (48) | 23 | 14 | |
| | | | | |
| Discipline of ICU admission | | | | 0.9 |
| Medicine (n; %) | 29 (3 8) | 19 (40) | 10 (36) | |
| General surgery (n; %) | 20 (26) | 13 (27) | 7 (25) | |
| Cardiac surgery (n; %) | 28 (36) | 17 (35) | 11 (39) | |
| Age (years) | 49 (39-60) | 49 (40-61) | 50 (38-60) | 0.8 |
| BMI (kg/m²) | 25 (22-28) | 25 (22-28) | 25 (22-28) | 0.6 |
| Indication for RRT | | | | |
| eGFR loss >30 % | 68 | 44 | 24 | 0.4 |
| Oliguria/anuria | 48 | 30 | 18 | 0.9 |
| Metabolic acidosis | 7 | 1 | 6 | 0.005* |
| Hyperkalemia | 6 | 2 | 4 | 0.1 |
| | | | | |
| SOFA score | 13 (11-16) | 12 (10-15) | 14 (13-18) | 0.05* |
| | | | | |
| RIFLE class | | | | 0.2 |
| Risk (n; %) | 5 (6) | 2 (4) | 3 (11) | |
| Injury (n; %) | 21 (27) | 16 (33) | 5 (18) | |
| Failure (n; %) | 50 (65) | 30 (63) | 20 (71) | |
| APACHE II score | 29 (25-34) | 28 (23-34) | 34 (29-38) | 0.4 |
| CRP (mg/L) | 128 (47-202) | 96 (48-205) | 141 (56-198) | 0.5 |
| MAP (mmHg) | 82 (71-94) | 84 (70-97) | 78 (73-86) | 0.3 |
| Heart rate (bpm) | 99 (88-109) | 92 (83-104) | 100 (96-117) | 0.6 |
| miR-16 | 1.9x10⁻⁴ (9.7x10⁻⁵-4.9x10⁻⁴) | 1.9x10⁻⁴ (9.4x10⁻⁵-4x10⁻⁴) | 2.2x10⁻⁴ (1.2x10⁻⁴- 1.2x10⁻³) | 0.9 |
| miR-210 | 4x10⁻⁵ (1.4x10⁻⁵- 1x10⁻⁴) | 3x10⁻⁵ (1x10⁻⁵ 7.6x10⁻⁵) | 7x10⁻⁵ (2.8x10⁻⁵-1.2x10⁻⁴) | 0.02* |
| miR-320 | 1x10⁻⁴ (5x10⁻⁵-3.4x10⁻⁴) | 1x10⁻⁴ (4x10⁻⁵-2.8x10⁻⁴) | 1.7x10⁻⁴ (8.3x10⁻⁵-4.1x10⁻⁴) | 0.03* |

These data show that no association between the microRNAs analysed and the APACHE II score or the SOFA score can be found. Further, no significant differences in concentrations of the microRNAs indicated in patients with or without sepsis (miR-16: p=0.3, miR-210: p=0.4; miR-320: p=0.6), surgery (miR-16: p=0.4; miR-210: p=0.7; miR-320: p=0.8), or shock (miR-16: p=0.5; miR-210: p=0.5; miR-320: p=0.4) were found. Similarly, the concentrations of the microRNAs analyzed did not differ between patients of the different intensive care units as assessed by ANOVA (miR-16: p=0.4; miR-210: p=0.3; miR-320: p=0.08). The concentrations of the microRNAs analysed did not correlate with serum creatinine levels at the start of renal replacement therapy (RRT) (miR-16: r=-0.14 p=0.2; miR-210: r=-0.3 p=0.8; miR-320: r=-0.4, p=0.8).

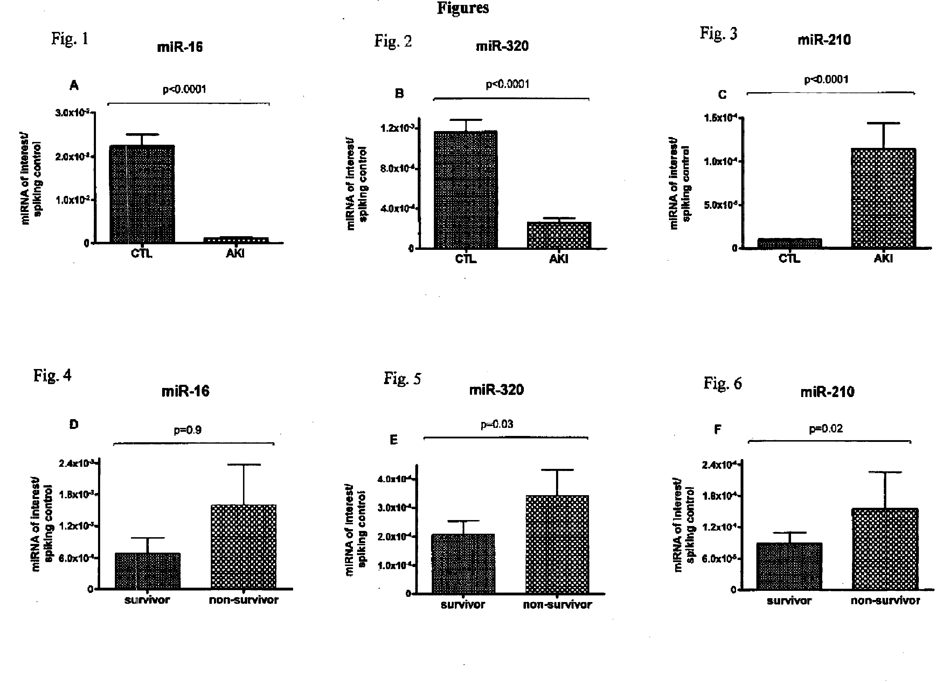

The analytical results for the AKI patients and healthy controls (CTL) of the determined concentration of miR-16 in serum is shown in Figure 1, for miR-320 in Figure 2, and for miR-210 in Figure 3. For the analysis, serum samples were spiked with recombinant miR-54 of Caenorhabditis elegans to 5 fmol/µL as an internal control miR, which was detected in parallel by the quantitative RT-PCR using the TaqMan method with specific reverse transcription primer, amplification primers, and detection oligonucleotide, to normalize for possible differences in the efficiency of the analytical method, including efficiency in RNA isolation from the serum. Spiked serum was used for RNA isolation with the MasterPure ^{(™)} RNA purification kit obtainable from Epicenter Biotechnologies.

Normalization of measurement results for the analyte microRNAs using the detected concentration of the miR-54 (SEQ ID NO 4: UACCCGUAAUCUUCAUAAUCCGAG) was done according to Fichtlscherer et al ("Circulating microRNAs in patients with coronary artery disease", Circ. Res. 677 - 684 (2010)). In short, the concentration of analyte miR, e.g. of miR-210, miR-320, and of miR-16 was calculated from the number of specific amplification products per reaction volume to obtain a calculated initial copy number of each analyte miR per initial sample volume, which initial copy number of analyte miR per initial sample volume was divided by the initial copy number concentration of added control miR, e.g. divided by the calculated concentration of control miR that was obtained from an added concentration of 5 fmol/µL serum, thus accounting for losses during RNA isolation.

Using this normalization of control miR-54 of C. elegans added to 5 fmol/µL initial sample volume, isolation of total RNA, RT-PCR using primer pairs specific for the analyte miR, e.g. for miR-210 and a primer pair specific for the control miR-54, the mean value of the concentration of miR-210 normalized by dividing the calculated initial concentration of miR-210 in the sample by the concentration of added control miR, added to a serum concentration of 5 fmol/µL serum, was determined to a value of 4.00098 x 10⁻⁵. Serum concentrations of miR-210 above a ratio of 4.00098 x 10⁻⁵ (initial concentration of miR-210 calculated from RT-PCR products) / (initial concentration calculated for control miR-54) in patients suffering from AKI indicate a worse prognosis for a period of 28 days, which was the observation period.

Standard curves of the correlation of the concentration of specific RT-PCR amplification products to the original concentration of the respective microRNA in the RNA were pre-determined with at least 4 dilutions of isolated total RNA containing the microRNA at a known concentration, each dilution differing by a factor of 10. Preferably, the known microRNA was added to a known concentration in the serum, and total RNA was isolated from serum, diluted to at least 2 dilution steps, and subjected to RT-PCR using a primer pair specific for the microRNA that was originally added to the serum.

Generally, the analytical method can include the step of measuring the concentration of a known housekeeping microRNA present in serum, and dividing the concentration of the analyte microRNA by the concentration of the housekeeping microRNA for normalization.

The results shown in Figures 1 to 3 demonstrate that in all the AKI patients and control individuals, it could be confirmed that lower than median concentrations of miR-16 and of miR-320 (both p < 0.001), and higher than median concentrations of miR-210 in patients with AKI in comparison to healthy controls were found.

The only significant association that was found were baseline concentrations of miR-16 (r=0.3, p=0.02), miR-210 (r=0.4, pp<0.001), and of miR-320 (r=0.4, pp<0.001) correlating with lactate levels, and baseline concentrations of miR-210 additionally was correlated with heart-rate (r=0.04, p<0.0001) baseline concentrations of miR-320 was correlated with heart-rate (r=0.4, p<0.0001), and noradrenalin dose (r=0.3, p=0.04).

Following the initial analysis, AKI patients underwent renal replacement therapy, and patients were grouped 4 weeks after the therapy in survivors and non-survivors. It was found that generally both these patient groups were comparable with respect to baseline demographics and to the proportion of RIFLE categories as shown in table 1. It was found that the group of survivors and the group of non-survivors only differed with regard to the proportion of patients with metabolic acidosis (p=0.005), and SOFA score (p=0.05). It was found that patients suffering from sepsis have a higher SOFA and higher APACHE II scores, which were deemed to result from more severe cardiovascular and respiratory impairment compared to the non-septic patients.

The analytical results for the AKI patients for survivors and non-survivors are shown in Figure 4 for concentration of miR-16, in Figure 5 for miR-320, and in Figure 6 for the concentration of miR-210. It is found that the concentration of miR-210 (p=0.02) and of miR-320 (p= 0.03) were significantly higher in non-survivors, while miR-16 did not show a difference between non-survivors and survivors.

An initial analysis by the univariate cox proportional hazards analysis was done to determine a possible relationship between circulating concentrations of microRNAs at initiation of RRT and mortality. It was found that in the initial AKI patients, concentrations of miR-16, age, gender, and body mass index were not significantly associated with survival, as shown in the following Table 2.

**Table 2: Univariate and multivariate Cox regression analysis for survival**

| | Univariate | | | Multivariate | | |
|---|---|---|---|---|---|---|
| Variables | HR | 95% CI | p-value | HR | 95% CI | p-value |
| miR-210 (log10) | 1.868 | 1.079 to 3.232 | 0.03* | 1.719 | 0.999 to 2.959 | 0.05* |
| miR-320 (log10) | 2.119 | 1.074 to 4.181 | 0.03* | n.s. | | |
| miR-16 (log10) | 1.299 | 0.753 to 2.242 | 0.3 | | | |
| Age (years) | 0.994 | 0.969 to 1.020 | 0.7 | | | |
| Body mass index (kg/m²) | 0.972 | 0.897 to 1.054 | 0.5 | | | |
| Gender (m/f) | 0.909 | 0.433 to 1.907 | 0.8 | | | |
| Sepsis (yes/no) | 2.257 | 1.071 to 4.753 | 0.03* | n.s. | | |
| Surgery (yes/no) | 2.024 | 0.947 to 4.324 | 0.07 | n.s. | | |
| SOFA score | 1.203 | 1.066 to 1.357 | 0.003* | 1.194 | 1.054 to 1.352 | 0.005* |
| APACHE II score | 1.055 | 1.007 to 1.106 | 0.03* | n.s. | | |

| | | | | | | |
|---|---|---|---|---|---|---|
| CI = confidence interval, HR = hazard ratio, n.s. = non-significant, * = p<0.1 | | | | | | |

From the data of Table 2, it was found that the variables major surgery, sepsis, APACHE II score, and SOFA score as well as concentrations of miR-210, and miR-320 display a prognostic significance at a 10% level. In multivariate analysis, it was found that only the SOFA score (p= 0.005), and miR-210 (p= 0.05) are independent predictors of survival. In ROC curve analysis miR-210 yielded an area under the ROC curve of 0.74 (standard error of the mean: 0.06; 95% confidence interval: 0.53 to 0.78; p= 0.03), while the concentration of miR-320 showed a similar level of significance (area under the ROC curve 0.7; standard error of the mean: 0.07; 95% confidence interval: 0.53 to 0.78; p= 0.03). The concentrations of miR-16 did not reach statistical significance (area under the ROC curve 0.55, standard error of the mean: 0.07, 95% confidence interval: 0.41 to 0.69, p=0.4). For comparison, the area under the curve of the SOFA score gave a value of 0.7 (standard error of the mean: 0.06, 95% confidence interval: 0.57 to 0.82, p=0.005).

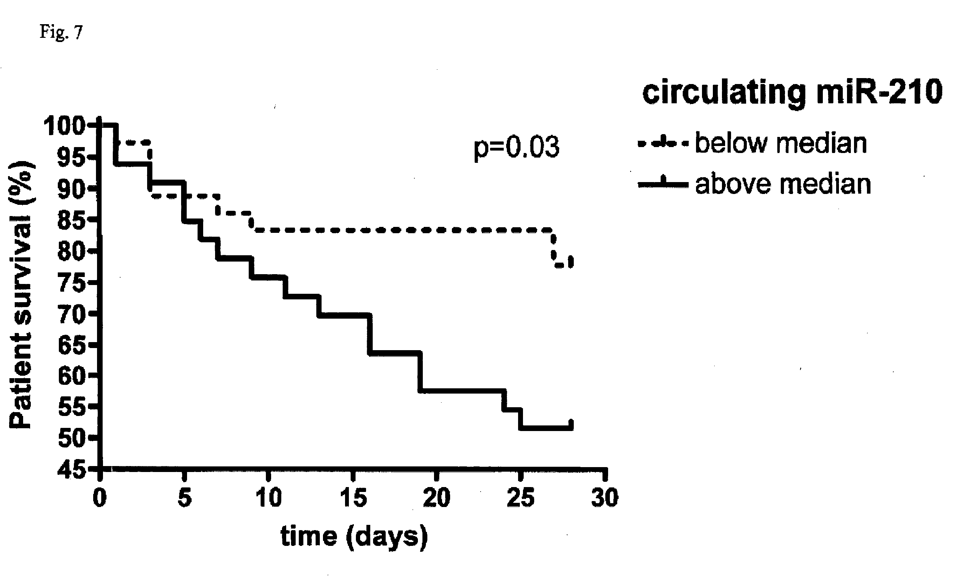

Figure 7 shows the Kaplan-Meier curve of survival at 4 weeks after initiation of RRT, stratified to miR-210 concentration above and below the median concentration value. The Log rank test confirmed the statistical significance for miR-210 (p=0.03), while miR-320 (p=0.08) and miR-16 (p=0.4) did not show statistical significance in this test.

### Example: Animal model of acute kidney injury

As an animal model, renal ischemia/reperfusion-injury (IRI) was induced in C57B1/6 mice by bilateral clamping of both renal pedicals. The animals were anesthetized with isoflurane and generally handled according to animal experiment guidelines. After median laparatomy, renal pedicals were bluntly dissected and a non-traumatic vascular clamp was applied for 27 min. At 4 weeks after induction of bilateral IRI animals were sacrificed and RNA was isolated from whole kidneys. Sham animals were used as controls. For in vivo real-time polymerase chain reaction (RT-PCR) analysis, RNA was extracted from renal tissue with Trizol reagent (Invitrogen). After reverse transcription of total RNA, qRT-PCR was performed with the Taqman MicroRNA assay (Applied Biosystems, ABI) according to the manufacturer's recommendations to detect the level of miR-210. miR-210 was normalized to the housekeeping small RNA snoRNA202 (SEQ ID NO 5). For comparison to control animals, miR-210 concentrations were calculated in relation to the concentration of the housekeeping snoRNA-202.

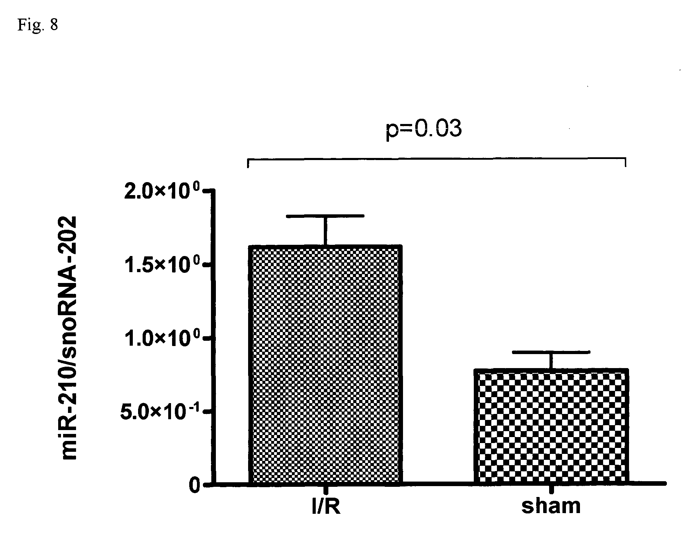

The result is shown in Figure 8, demonstrating that also in the animal experiment, the concentration of miR-210 was elevated in induced acute kidney injury. Further, the data show that elevated miR-210 level is indicative of renal tissue. Accordingly, it is at present concluded that the serum levels of miR-210 which have been found to be indicative of acute kidney injury in humans could directly originate from the kidney tissue.

## Claims

1. Method for analysis of kidney status in a sample of body fluid by determining the concentration of an analyte,
**characterized in that** the kidney status is acute kidney injury and the analyte is miR-210 and comprising adding a known microRNA as a control to the sample and analysing the concentration of the added control microRNA by parallel dectection with specificity for the control microRNA.

2. Method according to claim 1, **characterized by** additionally determining the concentration of at least one of the analytes of the group containing miR-16 and miR-320.

3. Method according to one of the preceding claims, **characterized in that** the control microRNA is a known concentration of miR-21 0, miR-320 and/or of miR-16 and/or of a microRNA of non-human origin.

4. Method according to one of the preceding claims, **characterized in that** the step of determining the concentration of the analyte comprises the step of contacting the sample of body fluid with a nucleic acid sequence which comprises a section that is reverse complementary to the analyte and detecting the concentration of nucleic acid hybrids formed of the nucleic acid sequence comprising the reverse complementary nucleic acid section.

5. Method according to one of the preceding claims, **characterized in that** the nucleic acid sequence containing the nucleic acid section which is reverse complementary to the analyte is immobilized on a carrier substrate.

6. Method according to one of the preceding claims, **characterized in that** the nucleic acid sequence containing the nucleic acid section which is reverse complementary to the analyte is labelled by a detectable chromophore or labelled by a combination of a detectable chromophore and a spaced-apart quencher to the chromophore.

7. Method according to one of the preceding claims, **characterized in that** the step of determining the concentration of the analyte comprises a reverse transcription reaction using an oligonucleotide as a primer that is specific for the analyte and a PCR reaction using two oligonucleotides as primers which are specific for the analyte.

8. Method according to claim 6, **characterized in that** the amplification product of the PCR reaction is specifically detected by hybridization with a labelled oligonucleotide containing the nucleic acid sequence of the analyte or containing a nucleotide sequence that is reverse complementary to the analyte.

9. Method according to one of the preceding claims, **characterized in that** the method comprises the step of
- adding a known control miRNA to a known concentration to the sample,
- isolating total RNA from the sample,
- carrying out RT-PCR on the RNA with specific primer pairs added for each of the analyte and for the control miRNA, respectively,
- calculating the original concentration of analyte by means of the standard curve, which contains the pre-determined correlation of the detected concentration of amplification products of the analyte to the original concentration of miRNA,
- calculating the original concentration of control miRNA by means of a pre-determined standard curve which contains the pre-determined correlation of the detected concentration of amplification products of the control miRNA to the original concentration of control miRNA, and
- forming the quotient of the calculated original concentration of analyte and the calculated concentration of control miRNA.

10. Method according to one of the preceding claims, **characterized in that** the method comprises the step of
- isolating total RNA from the sample,
- carrying out RT-PCR on the RNA with specific primer pairs added for each of the analyte and for a housekeeping RNA, respectively,
- calculating the original concentration of analyte by means of the standard curve, which contains the pre-determined correlation of the detected concentration of amplification products of the analyte to the original concentration of analyte miRNA,
- calculating the original concentration of housekeeping RNA by means of a pre-determined standard curve which contains the pre-determined correlation of the detected concentration of amplification products of the housekeeping RNA to the original concentration of housekeeping RNA, and
- forming the quotient of the calculated original concentration of analyte and the calculated concentration of housekeeping RNA.

11. Method according to one of the preceding claims, **characterized in that** the sample originates from a patient who has not been diagnosed with acute kidney injury.

12. Method according to one of claims 1 to 10, **characterized in that** the sample originates from a patient who has been diagnosed with acute kidney injury.

13. Method according to one of the preceding claims, **characterized by** comparing the detected concentration of the analyte to the concentration of the analyte in healthy persons.

14. Use of a device as an indicator substance which is specific for acute kidney injury in a method according to one of the preceding claims, the device containing a nucleic acid sequence containing the sequence of miR-210 and/or a nucleic acid section that is reverse complementary to miR-210

15. Use according to claim 14 as an indicator substance which is specific for kidney status, **characterized by** the device additionally containing a nucleic acid sequence containing a section that is identical to miR-16 and/or to miR-210, and/or a nucleotide section which is reverse complementary to miR-16 and/or reverse complementary to miR-210.

16. Use according to one of claims 14 to 15, **characterized in that** the nucleic acid sequence is immobilized on a carrier substrate.

## Patentansprüche

1. Verfahren zur Analyse des Nierenzustands in einer Probe von Körperflüssigkeit durch Bestimmen der Konzentration eines Analyten,
**dadurch gekennzeichnet, dass** der Nierenzustand akutes Nierenversagen ist und der Analyt miR-210 ist und das Zugeben einer bekannten microRNA als eine Kontrolle zu der Probe umfasst, sowie das Analysieren der Konzentration der zugegebenen Kontroll- mikroRNA durch parallelen Nachweis mit Spezifität für die Kontroll-mikroRNA.

2. Verfahren nach Anspruch 1, **gekennzeichnet durch** das zusätzliche Bestimmen der Konzentration von wenigstens einem der Analyten aus der Gruppe, die miR-16 und miR-320 enthält.

3. Verfahren nach einem der voranstehenden Ansprüche, **dadurch gekennzeichnet, dass** die Kontroll-mikroRNA eine bekannte Konzentration von miR-210, miR-320 und/oder von miR-16 und/oder einer microRNA nicht-menschlichen Ursprungs ist.

4. Verfahren nach einem der voranstehenden Ansprüche, **dadurch gekennzeichnet, dass** der Schritt des Bestimmens der Konzentration des Analyten den Schritt des Kontaktierens der Probe Körperflüssigkeit mit einer Nukleinsäuresequenz umfasst, die einen Abschnitt umfasst, der zum Analyten revers komplementär ist und Nachweisen der Konzentration der Nukleinsäurehybride, die aus der Nukleinsäuresequenz gebildet sind, die den revers komplementären Nukleinsäureabschnitt umfasst.

5. Verfahren nach einem der voranstehenden Ansprüche, **dadurch gekennzeichnet, dass** die Nukleinsäuresequenz, die den Nukleinsäureabschnitt enthält, der zum Analyten revers komplementär ist, auf einem Trägersubstrat immobilisiert ist.

6. Verfahren nach einem der voranstehenden Ansprüche, **dadurch gekennzeichnet, dass** die Nukleinsäuresequenz, die den Nukleinsäureabschnitt enthält, der zum Analyten revers komplementär ist, mit einem nachweisbaren Chromophor markiert ist, oder durch eine Kombination eines nachweisbaren Chromophors und einem beabstandeten Quencher für den Chromophor markiert ist.

7. Verfahren nach einem der voranstehenden Ansprüche, **dadurch gekennzeichnet, dass** der Schritt des Bestimmens der Konzentration des Analyten eine reverse Transkriptionsreaktion unter Verwendung eines Oligonukleotids als einen Primer umfasst, der für den Analyten spezifisch ist und eine PCR-Reaktion unter Verwendung von zwei Oligonukleotiden als Primer, die für den Analyten spezifisch sind.

8. Verfahren nach Anspruch 6, **dadurch gekennzeichnet, dass** das Amplifikationsprodukt der PCR-Reaktion durch Hybridisierung mit einem markierten Oligonukleotid spezifisch nachgewiesen wird, das die Nukleinsäuresequenz des Analyten enthält oder eine Nukleotidsequenz enthält, die zu dem Analyten revers komplementär ist.

9. Verfahren nach einem der voranstehenden Ansprüche, **dadurch gekennzeichnet, dass** das Verfahren die Schritte umfasst von
- Zugeben einer bekannten Kontroll-miRNA zu einer bekannten Konzentration der Probe,
- Isolieren der Gesamt-RNA aus der Probe,
- Ausführen einer RT-PCR zu der RNA mit spezifischen Primerpaaren, die für jeweils den Analyten bzw. für die Kontroll-miRNA zugegeben sind,
- Berechnen der ursprünglichen Konzentration des Analyten mittels einer Standardkurve, die die vorbestimmte Beziehung der nachgewiesenen Konzentration von Amplifikationsprodukten des Analyten zur ursprünglichen Konzentration der miRNA enthält,
- Berechnen der ursprünglichen Konzentration der Kontroll-miRNA mittels einer vorbestimmten Standardkurve, die die vorbestimmte Beziehung der nachgewiesenen Konzentration der Amplifikationsprodukte der Kontroll-miRNA zur ursprünglichen Konzentration der Kontroll-miRNA enthält und
- Bilden des Quotienten der berechneten ursprünglichen Konzentration des Analyten und der berechneten Konzentration der Kontroll-miRNA.

10. Verfahren nach einem der voranstehenden Ansprüche, **dadurch gekennzeichnet, dass** das Verfahren die Schritte umfasst von
- Isolieren der Gesamt-RNA aus der Probe,
- Durchführen von RT-PCR mit der RNA mit spezifischen Primerpaaren, die für jeweils den Analyten bzw. eine Haushalts-RNA zugegeben sind,
- Berechnen der ursprünglichen Konzentration des Analyten mittels der Standardkurve, die eine vorbestimmte Beziehung der nachgewiesenen Konzentration von Amplifikationsprodukten des Analyten zur ursprünglichen Konzentration der Analyten-miRNA enthält,
- Berechnen der ursprünglichen Konzentration der Haushalts-RNA mittels einer vorbestimmten Standardkurve, die die vorbestimmte Beziehung der nachgewiesenen Konzentration von Amplifikationsprodukten der Haushalts-RNA zur ursprünglichen Konzentration der Haushalts-RNA enthält und
- Bilden des Quotienten der berechneten ursprünglichen Konzentration des Analyten und der berechneten Konzentration der Haushalts-RNA.

11. Verfahren nach einem der voranstehenden Ansprüche, **dadurch gekennzeichnet, dass** die Probe von einem Patienten stammt, für den nicht das akute Nierenversagen diagnostiziert wurde.

12. Verfahren nach einem der Ansprüche 1 bis 10, **dadurch gekennzeichnet, dass** die Probe von einem Patienten stammt, für den das akute Nierenversagen diagnostiziert wurde.

13. Verfahren nach einem der voranstehenden Ansprüche, **kennzeichnet durch** das Vergleichen der nachgewiesen Konzentration des Analyten zur Konzentration des Analyten in gesunden Personen.

14. Verwendung einer Vorrichtung als eine Indikatorverbindung, die für akutes Nierenversagen spezifisch ist, in einem Verfahren gemäß einem der voranstehenden Ansprüche, wobei die Vorrichtung eine Nukleinsäuresequenz enthält, die die Sequenz von miR-210 und/oder einen Nukleinsäureabschnitt enthält, der zu miR-210 revers komplementär ist.

15. Verwendung nach Anspruch 14 als eine Indikatorverbindung, die für den Nierenzustand spezifisch ist, **dadurch gekennzeichnet, dass** die Vorrichtung zusätzlich eine Nukleinsäuresequenz enthält, die einen Abschnitt enthält, der zu miR-16 und/oder zu miR-210 identisch ist, oder einen Nukleotidabschnitt, der zu miR-16 revers komplementär ist und/oder zu miR-210 revers komplementär ist.

16. Verwendung nach einem der Ansprüche 14 bis 15, **dadurch gekennzeichnet, dass** die Nukleinsäuresequenz auf einem Trägersubstrat immobilisiert ist.

## Revendications

1. Procédé pour l'analyse d'un état rénal dans un échantillon de liquide corporel en déterminant la concentration d'une substance à analyser, **caractérisé en ce que** l'état rénal est une insuffisance rénale aiguë et que la substance à analyser est miR-210 et comprend l'ajout d'un microARN connu comme témoin à l'échantillon et l'analyse de la concentration du microARN témoin ajouté par détection parallèle de la spécificité du microARN témoin.

2. Procédé selon la revendication 1, **caractérisé par** la détermination, en sus, de la concentration d'au moins un des substances à analyser du groupe contenant miR-16 et miR-320.

3. Procédé selon l'une quelconque des revendications précédentes, **caractérisé en ce que** le microARN témoin est une concentration connue de miR-210, de miR-320 et/ou de miR-16 et/ou d'un microARN d'origine non humaine.

4. Procédé selon l'une quelconque des revendications précédentes, **caractérisé en ce que** l'étape de détermination de la concentration de la substance à analyser comprend l'étape de mise en contact de l'échantillon de liquide corporel avec une séquence d'acide nucléique comprenant une section inversement complémentaire à la substance à analyser et la détection de la concentration des hybrides d'acide nucléique formés de la séquence d'acide nucléique comprenant la section d'acide nucléique inverse complémentaire.

5. Procédé selon l'une quelconque des revendications précédentes, **caractérisé en ce que** la séquence d'acide nucléique contenant la section d'acide nucléique inversement complémentaire à la substance à analyser est immobilisée sur un substrat support.

6. Procédé selon l'une quelconque des revendications précédentes, **caractérisé en ce que** la séquence d'acide nucléique contenant la section d'acide nucléique inversement complémentaire à la substance à analyser est marquée par un chromophore détectable ou marquée par une combinaison de chromophore détectable et d'extincteur séparé du chromophore.

7. Procédé selon l'une quelconque des revendications précédentes, **caractérisé en ce que** l'étape de détermination de la concentration de la substance à analyser comprend une réaction de transcription inverse utilisant un oligonucléotide servant d'amorce spécifique à la substance à analyser et une réaction PCR utilisant deux oligonucléotides comme amorces spécifiques à la substance à analyser.

8. Procédé selon la revendication 6, **caractérisé en ce que** le produit d'amplification de la réaction PCR est détecté de façon spécifique par hybridation avec un oligonucléotide marqué contenant la séquence d'acide nucléique de la substance à analyser ou contenant une séquence de nucléotide inversement complémentaire à la substance à analyser.

9. Procédé selon l'une quelconque des revendications précédentes, **caractérisé en ce que** le procédé comprend l'étape de :
- ajout d'un miARN témoin connu en concentration connue à l'échantillon ;
- isolement de l'ensemble de l'ARN du reste de l'échantillon ;
- réalisation de la réaction en chaîne par polymérase RT-PCR sur l'ARN pourvu de paires d'amorces spécifiques ajoutées à chaque substance à analyser et au miARN témoin, respectivement ;
- calcul de la concentration d'origine de la substance à analyser au moyen de la courbe standard contenant la corrélation prédéterminée de la concentration détectée des produits d'amplification de la substance à analyser sur la concentration d'origine de miARN ;
- calcul de la concentration d'origine de miARN témoin au moyen d'une courbe standard prédéterminée contenant la corrélation prédéterminée de la concentration détectée des produits d'amplification du miARN témoin sur la concentration d'origine de miARN témoin ; et
- formation du quotient de la concentration d'origine calculée de la substance à analyser et de la concentration calculée de miARN témoin.

10. Procédé selon l'une quelconque des revendications précédentes, **caractérisé en ce que** le procédé comprend l'étape de :
- isolement de l'ensemble de l'ARN du reste de l'échantillon ;
- réalisation de la réaction en chaîne par polymérase RT-PCR sur l'ARN avec les paires d'amorces spécifiques ajoutées à chaque substance à analyser et à un ARN constitutif, respectivement ;
- calcul de la concentration d'origine de substance à analyser au moyen de la courbe standard contenant la corrélation prédéterminée de la concentration détectée des produits d'amplification de la substance à analyser sur la concentration d'origine de la substance à analyser de miARN ;
- calcul de la concentration d'origine d'ARN constitutif au moyen d'une courbe standard prédéterminée contenant la corrélation prédéterminée de la concentration détectée des produits d'amplification de l'ARN constitutif sur la concentration d'origine de l'ARN constitutif ; et
- formation du quotient de la concentration d'origine calculée de la substance à analyser et de la concentration calculée de l'ARN constitutif.

11. Procédé selon l'une quelconque des revendications précédentes, **caractérisé en ce que** l'échantillon provient d'un patient chez lequel aucune insuffisance rénale aiguë n'a été diagnostiquée.

12. Procédé selon l'une quelconque des revendications 1 à 10, **caractérisé en ce que** l'échantillon provient d'un patient chez lequel une insuffisance rénale aiguë a été diagnostiquée.

13. Procédé selon l'une quelconque des revendications précédentes, **caractérisé en ce que** l'on compare la concentration détectée de la substance à analyser sur la concentration de la substance à analyser chez des personnes saines.

14. Utilisation d'un dispositif servant de substance indicatrice spécifique à l'insuffisance rénale aiguë dans un procédé selon l'une quelconque des revendications précédentes, le dispositif contenant une séquence d'acide nucléique contenant la séquence de miR-210 et/ou une section d'acide nucléique inversement complémentaire à miR-210.

15. Utilisation selon la revendication 14 d'une substance indicatrice spécifique à un état rénal, **caractérisée en ce que** le dispositif contient en outre une séquence d'acide nucléique contenant une section identique à miR-16 et/ou à miR-210, et/ou une section de nucléotide inversement complémentaire à miR-16 et/ou inversement complémentaire à miR-210.

16. Utilisation selon l'une quelconque des revendications 14 à 15, **caractérisée en ce que** la séquence d'acide nucléique est immobilisée sur un substrat support.


### SEQUENCE LISTING

<110> Medizinische Hochschule Hannover
<120> Device and method for analysis of kidney failure
<130> M1038-N-EP
<150> EPEP11153273
   <151> 2011-02-03
<160> 5
<170> BiSSAP 1.0
<210> 1
   <211> 22
   <212> RNA
   <213> Homo sapiens
<220>
   <221> source
   <222> 1..22
   <223> /mol_type="RNA" /organism="Homo sapiens"
<220>
   <221> ncRNA
   <222> 1..22
   <223> /ncRNA_class="miRNA"
<400> 1
   cugugcgugu gacagcggcu ga 22
<210> 2
   <211> 22
   <212> RNA
   <213> Homo sapiens
<220>
   <221> source
   <222> 1..22
   <223> /mol_type="RNA" /organism="Homo sapiens"
<220>
   <221> ncRNA
   <222> 1..22
   <223> /ncRNA_class="miRNA"
<400> 2
   aaaagcuggg uugagagggc ga 22
<210> 3
   <211> 22
   <212> RNA
   <213> Homo sapiens
<220>
   <221> source
   <222> 1..22
   <223> /mol_type="RNA" /organism="Homo sapiens"
<220>
   <221> ncRNA
   <222> 1..22
   <223> /ncRNA_class="miRNA"
<400> 3
   uagcagcacg uaaauauugg cg 22
<210> 4
   <211> 24
   <212> RNA
   <213> Caenorhabditis elegans
<220>
   <221> source
   <222> 1..24
   <223> /mol_type="RNA" /organism="Caenorhabditis elegans"
<220>
   <221> ncRNA
   <222> 1..24
   <223> /ncRNA_class="miRNA"
<400> 4
   uacccguaau cuucauaauc cgag 24
<210> 5
   <211> 48
   <212> DNA
   <213> Mus musculus
<220>
   <221> source
   <222> 1..48
   <223> /mol_type="DNA" /organism="Mus musculus"
<220>
   <221> ncRNA
   <222> 1..48
   <223> /ncRNA_class="snoRNA"
<400> 5
   gctgtactga cttgatgaaa gtacttttga acccttttcc atctgatg 48